# EUROPÄISCHE PATENTSCHRIFT

(11) **EP 0 493 662 B1**
(45) Veröffentlichungstag und Bekanntmachung des Hinweises auf die Patenterteilung: **06.12.1995**
(21) Anmeldenummer: 91118805.0
(22) Anmeldetag: 05.11.1991
(51) Int. Cl.: A61K 38/44

(54) **Verwendung von Superoxiddismutasen für die Herstellung von Arzneimitteln zur Prophylaxe und/oder Behandlung von Organversagen bei Risikopatienten mit Polytrauma als Unfallfolge**
Use of superoxide dismutase for the preparation of drugs for the prophylaxis and/or treatment of organ failure in risk patients having a polytrauma due to an accident
Utilisation de superoxyde dismutases pour la préparation de médicaments pour la prophylaxie et/ou le traitement de la défaillance d'organes de patients à risques ayant un traumatisme multiple causé par un accident

(30) Priorität: 04.12.1990 DE 4038563
(43) Veröffentlichungstag der Anmeldung: 08.07.1992
(73) Patentinhaber: Grünenthal GmbH, D-52078 Aachen (DE)
(72) Erfinder: Schüttler, Achim, Dr., W-5100 Aachen (DE); Flohé, Leopold, Prof. Dr., W-3340 Wolfenbüttel-Halchter (DE); Bühren, Volker, Dr., W-6650 Homburg/Saar (DE); Marzi, Ingo, Dr., W-6650 Homburg/Saar (DE); Trentz, Otmar, Prof. Dr., CH-8006 Zürich (CH)

(56) Entgegenhaltungen:
- EP-A- 0 189 182
- CIRCULATORY SHOCK, vol. 31, 1990, Abstr. Nr.8 (Schlag et al.)
- CIRCULATORY SHOCK, Band 30, Nr. 2, Februar 1990, NEW YORK, USA, Seiten 97-106; J. SCHNEIDER et al.: "Effects of recombinant human superoxide dismutase on increased lung vascular permeability and respiratory disorder in endotoxemic rats."
- TRANSPLANTATION PROCEEDINGS, Band 22, Nr. 5, Oktober 1990, NEW YORK, USA, Seiten 2224-2225; H. SCHNEEBERGER et al.: "Prevention of acute renal failure after kidney transplantation by treatment with rh-SOD: Interim analysis of a double-blind placebo-controlled trial."

## Beschreibung

Multi-Organversagen stellt die häufigste Todesursache bei denjenigen Patienten mit Polytrauma als Unfallfolge dar, die die ersten 24 Stunden nach der chirurgischen Primärversorgung überleben. Die Pathomechanismen, die zu dieser lebensbedrohlichen Komplikation führen, sind bisher ungeklärt. Eine gezielte Therapie oder auch Prophylaxe dieser Komplikation war daher bisher nicht möglich.

Bei einer Sepsis und dem nachfolgenden Organversagen werden die klinischen Zeichen fast ausschließlich auf das Eindringen von Mikroorganismen und die Freisetzung von Endotoxinen zurückgeführt. Dazu wurde als Erklärung vorgeschlagen, daß durch Endotoxin eine Aktivierung des Komplementsystems erfolgt, die zur Bildung von zahlreichen schädigenden Mediatoren, u. a. Sauerstoffradikalen, führt. Trotz bemerkenswerter Fortschritte in der Grundlagenforschung hinsichtlich des septischen Schocks ist aber nach wie vor unklar, welche Bedeutung den Sauerstoffradikalen neben anderen Mediatoren zukommt. Verschiedene experimentelle Untersuchungen zeigten jedoch bei Sepsismodellen eine positive Wirkung der Verabreichung von Superoxiddismutasen sowohl in Bezug auf den Verlauf des Krankheitsbildes als auch bezüglich der Letalität.

Demgegenüber erwiesen sich in tierexperimentellen Traumamodellen Superoxiddismutasen weder bei der Ratte noch bei Primaten als wirksam (Circ. Shock 31, Abstract Nr. 8; 1990). Ob Sauerstoffradikale neben den übrigen Mediatoren beim unfallbedingten Polytrauma eine wesentliche Rolle spielen, war daher offen und eher unwahrscheinlich.

Überraschenderweise wurde nun in klinischen Studien gefunden, daß nach der Verabreichung von hohen Dosen von Superoxiddismutase an Patienten mit Polytrauma als Unfallfolge eine signifikant positive Wirkung in Bezug auf die Entwicklung posttraumatischer lebensbedrohlicher Komplikationen einschließlich einfachen und multiplen Organversagens festzustellen war, wobei keine Nebenwirkungen, die auf die hohe Superoxiddismutasedosierung über mehrere Tage hätten zurückgeführt werden können, auftraten. Dieser positive Effekt mag daraus resultieren, daß die Konsequenzen der vielen Verletzungen nach einem derartigen Polytrauma als ein wiederholt auftretendes Reperfusionssyndrom (bekannt als Quelle von Sauerstoffradikalen) aufgefaßt werden können. Gegenstand der Erfindung ist daher die Verwendung von Superoxiddismutasen für die Herstellung von Arzneimitteln zur Prophylaxe und/oder Behandlung von Organversagen bei Risikopatienten mit Polytrauma als Unfallfolge.

Superoxiddismutasen sind natürlich vorkommende Metalloproteine, die in der Lage sind, Superoxidradikale zu dem weniger toxischen Wasserstoffperoxid zu dismutieren. Im allgemeinen enthalten die Enzyme ein oder zwei Metallkationen wie diejenigen von Eisen, Mangan, Kupfer, Zink, Cadmium, Kobalt oder Quecksilber. Das menschliche Enzym sowie die meisten von Säugern stammenden Superoxiddismutasen enthalten eine Kombination von Kupfer- und Zinkionen. Die menschliche Kupfer-/Zink-Superoxiddismutase besteht aus zwei gleichen Untereinheiten mit je 153 Aminosäureresten, und sie enthält je Untereinheit ein Kupfer- und ein Zinkatom. Das dimere Enzym hat ein Molekulargewicht von 32.000 D. Es ist aber auch eine tetramere Kupfer-/Zink-Superoxiddismutase eukaryotischen Ursprungs mit einem Molekulargewicht von 130.000 D beschrieben worden.

Eine Mangansuperoxiddismutase eukaryotischen Ursprungs ist ein Tetrameres mit einem Molekulargewicht von 80.000 D, und Eisen- und Manganformen bakteriellen Ursprungs sind Dimere mit Molekulargewichten von etwa 40.000 Dalton.

Bekannte Verfahren zur Gewinnung von Superoxiddismutasen gehen z. B. von Erythrozyten, Leber oder anderen Geweben aus (vgl. z. B. deutsche Patente 1 924 230 und 2 259 405 sowie deutsche Offenlegungsschrift 2 259 404 oder europäische Patentschrift 19 477). Während z. B. menschliche Superoxiddismutase N-terminal acetyliert ist, weisen Superoxiddismutasen aus einer Anzahl anderer natürlicher Vorkommen dieses Merkmal nicht auf.

Alternativ können Superoxiddismutasen auch aus kultivierten transformierten Zellinien gewonnen werden, wie dies beispielsweise in der internationalen Patentanmeldung WO 87/01387 beschrieben wurde.

Die Herstellung von Superoxiddismutasen aus transformierten Bakterien oder Hefen wurde beispielsweise in der DE-OS 36 39 725, in der europäischen Patentschrift 180 964 sowie in den europäischen Patentanmeldungen 172 577 und 284 105 beschrieben. Schließlich seien noch die Verfahren zur Gewinnung von Superoxiddismutasen mit Hilfe der rekombinanten DNA-Technologie in Hefen erwähnt, die beispielsweise in der europäischen Patentanmeldung 138 111 sowie in der europäischen Patentanmeldung 213 628 beschrieben wurden. Bei dem Verfahren der europäischen Patentanmeldung 138 111 wird menschliche Superoxiddismutase in der N-terminal acetylierten Form erhalten.

Eine nach den Angaben der europäischen Patentanmeldung 0 138 111 in transformierten Hefezellen gewonnene Kupfer-/Zink-Superoxiddismutase, die bei den weiter unten beschriebenen klinischen Untersuchungen eingesetzt wurde, zeigte eine minimale spezifische Aktivität von 3.000 Einheiten/mg (bestimmt entsprechend J. Biol. Chem. 244, Seiten 6049 bis 6055; 1969). Ihre Aminosäuresequenz ist identisch mit der von Kupfer-/Zink-Superoxiddismutase natürlichen menschlichen Ursprungs, die z. B. in Hoppe-Seyler's Z. Physiol. Chem. 364, Seiten 675 bis 690 (1983) beschrieben wurde.

Superoxiddismutasen als Wirkstoff enthaltende Arzneimittel bzw. pharmazeutische Zubereitungs- oder Anwendungsformen (für andere als die hier genannten Indikationen) sind z. B. aus der US-Patentschrift 3 637 640 oder aus den europäischen Patentanmeldungen 172 577, 295 826 sowie 342 620 bereits bekannt. Alle diese Arzneimittelformen kommen auch für die erfindungsgemäße Verwendung von Superoxiddismutasen für die Herstellung von Arzneimitteln für die definierten weiteren Indikationen in Betracht. Für die Infusionstherapie ist insbesondere eine durch Gefriertrocknung erhaltene sterile pyrogenfreie, gegebenenfalls durch Kohlehydrate stabilisierte Superoxiddismutasezubereitung geeignet.

Vorzugsweise kommen zur intravenösen Infusion geeignete Superoxiddismutasezubereitungen für die Prophylaxe und/oder Behandlung von Organversagen bei Risikopatienten mit Polytrauma als Unfallfolge in Betracht. Im allgemeinen wird die Superoxiddismutase dem Patienten durch intravenöse Infusion in einer Dosierung verabreicht, die konstante und ausreichend hohe Plasmaspiegel während mehrerer Tage gewährleistet. Die Behandlung sollte sobald wie möglich nach dem Unfall bzw. der chirurgischen Primärversorgung einsetzen. Dabei sollte die Dosierung zwischen etwa 1 g und etwa 15 g pro Tag liegen. Man erzielt damit Plasmaspiegel zwischen 30.000 und 1.000.000 Einheiten Superoxiddismutase pro Liter Plasma.

Die Dauer der Infusionstherapie ist abhängig von dem speziellen Krankheitsbild und dem Allgemeinzustand des Patienten. Sie liegt im Ermessen des behandelnden Arztes, wobei aber im allgemeinen die Applikation für mindestens 2 Tage erfolgen soll und für einen Zeitraum für bis zu 10 Tagen durchgeführt werden kann.

Die überraschende durch Superoxiddismutase-Infusion zu erzielende signifikante Verbesserung in Bezug auf die Entwicklung posttraumatischer lebensbedrohlicher Komplikationen einschließlich der Vermeidung eines Organversagens bei Patienten mit Polytrauma als Unfallfolge konnte mit den im folgenden beschriebenen klinischen Studien gezeigt werden:
24 Patienten wurden in einer randomisierten, placebokontrollierten Doppel-Blind-Studie in parallele Gruppen aufgenommen. Die Einschlußkriterien waren:
- Polytraumapatienten (älter als 18 Jahre) mit einem Schweregrad nach "Injury Severity Score (ISS)" (vgl. Greenspan et al., J. Trauma 25 (1985) 60 bis 64) von ≧ 27
- Therapiebeginn nicht später als 48 Stunden nach dem Unfallgeschehen (d. h. dem initialen Trauma).

Die Patienten hatten bei Verkehrs- oder Betriebsunfällen multiple Frakturen und Weichteilverletzungen erlitten. Ausgeschlossen von der Studie wurden Patienten, bei denen ein Schädel-Hirn-Trauma im Vordergrund der Verletzungen, stand.

Die Infusionstherapie wurde sobald wie möglich nach der chirurgischen Primärversorgung begonnen. Es wurden 3 g der rekombinanten Superoxiddismutase pro Tag für einen Zeitraum von 5 Tagen verabreicht. Der gesamte Beobachtungszeitraum betrug 14 Tage, während dessen kontinuierlich klinische und biochemische Parameter erhoben wurden. Diese Erhebung von Parametern richtete sich wesentlich nach ihrer Notwendigkeit für die Analyse mittels des in der Literatur beschriebenen "multiple-organe-failure-score (MOF-Scores)" nach Goris et al. (Arch. Surg. 120, Seiten 1109 bis 1115; 1985). Zusätzlich wurden biochemische Parameter erhoben, die zur weiteren Aufklärung der zugrundeliegenden Pathomechanismen des posttraumatischen Organversagens von Bedeutung sein können, wie z. B. Elastase-plasmaspiegel. Wie sich aus einer Arbeit in Deutschem Ärzteblatt 87, Seiten 952 bis 956 (1990) ergibt, korrelieren die Plasmaspiegel der Granulozyten-Elastase mit der Inzidenz posttraumatischer Komplikationen bei Polytraumapatienten. Ein Elastasespiegel von 85 »g/l am 5. Tag nach dem Unfallgeschehen stellt danach eine Diskriminierungsgrenze dar, oberhalb deren eine hohe Inzidenz von Komplikationen einschließlich Organversagen vorliegt.

Bei der durchgeführten klinischen Studie betrugen die Elastasespiegel am 5. Tag nach dem Unfall (also an Tag 6 der Studie) für die mit Superoxiddismutase behandelte Patientengruppe 57,9 ± 5,7 »g/l, während sie für die Placebogruppe 112 ± 23,7 »g/l betrugen. Die Anzahl der Patienten mit Elastasespiegeln oberhalb und unterhalb des Diskriminierungspunktes von 85 »g/l sind in der folgenden Tabelle zusammengestellt ("SOD" = Superoxiddismutase).

| Elastasespiegel am Tag 1 | | |
|---|---|---|
| | < 85 »g/1 | > 85 »g/1 |
| SOD | 1 | 11 |
| Placebo | 2 | 10 |

| Elastasespiegel am Tag 6 | | |
|---|---|---|
| | < 85 »g/1 | > 85 »g/1 |
| SOD | 11 | 1* |
| Placebo | 5 | 7 |

| | | |
|---|---|---|
| * Patient mit Elastasespiegel von 86 »g/1 | | |

Ein ähnlicher Unterschied wurde auch für das Akutphase-Protein CRP (C-reaktives Protein) mit 104 ± 16 mg/ml in der mit Superoxiddismutase behandelten Patientengruppe gegenüber 160 ± 19 mg/ml in der Placebogruppe gefunden.

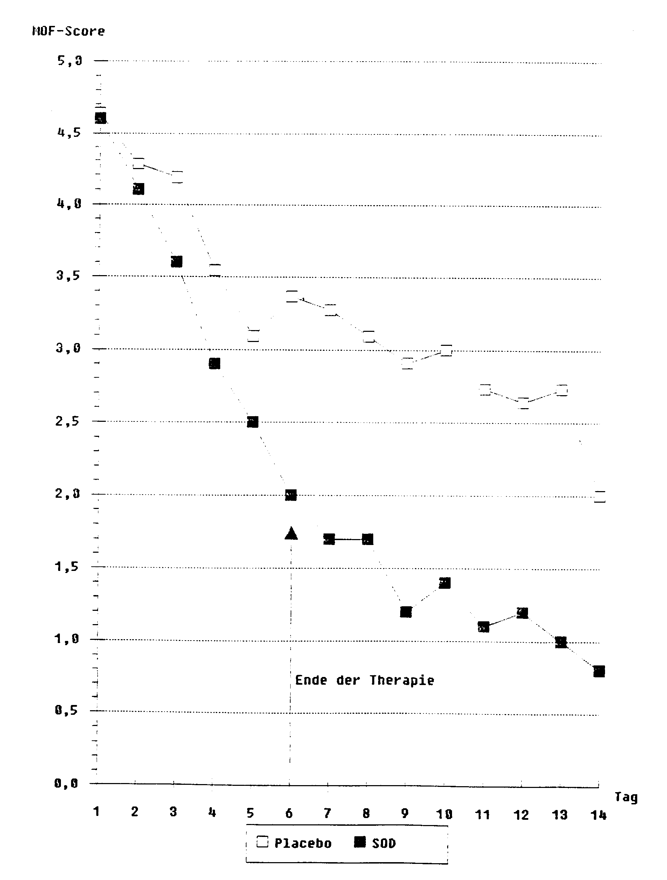

Der allgemeine klinische Eindruck bezüglich der Schwere der posttraumatischen Komplikationen läßt sich mittels des bereits erwähnten MOF-Scores nach Goris et al. darstellen. Die Auswertung der MOF-Scores zeigt deutlich eine signifikant bessere Organfunktion bei den mit Superoxiddismutase behandelten Patienten im Vergleich zu denen der Placebogruppe, wie sich aus der beigefügten Abbildung ergibt, in der niedrigere Score-Werte eine bessere Organfunktion anzeigen. Die Auswertung der MOF-Scores ergibt also ein ähnliches Bild wie die der Labordaten, d. h. daß unter der Behandlung mit Superoxiddismutase bei Patienten mit unfallbedingtem Polytrauma eine signifikant positive Wirkung in Bezug auf die Entwicklung posttraumatischer Komplikationen beobachtet und nachgewiesen werden konnte.

## Patentansprüche

1. Verwendung von Superoxiddismutasen für die Herstellung von Arzneimitteln zur Prophylaxe und/oder Behandlung eines Organversagens bei Risikopatienten mit Polytrauma als Unfallfolge.

2. Verwendung von Superoxiddismutasen für die Herstellung von Arzneimitteln gemäß Anspruch 1, dadurch gekennzeichnet, daß diese Arzneimittel zur intravenösen Infusion geeignet sind.

3. Verwendung von Superoxiddismutasen für die Herstellung von Arzneimitteln gemäß Anspruch 2 zur intravenösen Infusion, dadurch gekennzeichnet, daß diese Arzneimittel eine durch Gefriertrocknung erhaltene sterile pyrogenfreie, gegebenenfalls durch Kohlehydrate stabilisierte Superoxiddismutasezubereitung darstellen.

4. Verwendung einer Superoxiddismutase für die Herstellung von Arzneimitteln gemäß Ansprüchen 1 bis 3, dadurch gekennzeichnet, daß natürliche oder rekombinante menschliche Kupfer/Zink-Superoxiddismutase zur Anwendung gelangt.

## Claims

1. Use of superoxide dismutases for the production of pharmaceutical preparations for the prophylaxis and/or treatment of organ failure in high risk patients with multiple trauma as a consequence of accidents.

2. Use of superoxide dismutases for the production of pharmaceutical preparations according to claim 1, characterised in that these pharmaceutical preparations are suitable for intravenous infusion.

3. Use of superoxide dismutases for the production of pharmaceutical preparations according to claim 2 for intravenous infusion, characterised in that these pharmaceutical preparations constitute a sterile, pyrogen-free, optionally carbohydrate-stabilised superoxide dismutase preparation obtained by freeze drying.

4. Use of a superoxide dismutase for the production of pharmaceutical preparations according to claims 1 to 3, characterised in that natural or recombinant human copper/zinc superoxide dismutase is used.

## Revendications

1. Utilisation de superoxyde-dismutases pour la préparation de médicaments destinés à la prophylaxie et/ou au traitement d'une défaillance d'organes chez des patients à risques atteints de traumatismes multiples à la suite d'un accident.

2. Utilisation de superoxyde-dismutases pour la préparation de médicaments suivant la revendication 1, caractérisée en ce que ces médicaments conviennent pour une perfusion intraveineuse.

3. Utilisation de superoxyde-dismutases pour la préparation de médicaments selon la revendication 2 pour perfusion intraveineuse, caractérisée en ce que ces médicaments constituent une préparation de superoxyde-dismutases apyrogène stérile obtenue par lyophilisation, éventuellement stabilisée par des glucides.

4. Utilisation d'une superoxyde-dismutase pour la préparation de médicaments selon les revendications 1 à 3, caractérisée en ce qu'on emploie une superoxyde-dismutase humaine au cuivre/zinc, naturelle ou recombinante.
